# EUROPEAN PATENT SPECIFICATION

(11) **EP 1 784 136 B1**
(45) Date of publication and mention of the grant of the patent: **16.03.2011**
(21) Application number: 04761883.0
(22) Date of filing: 30.08.2004
(51) Int. Cl.: A61B 17/88, A61F 2/46

(54) **Hand-held motorized injection device with haptic feedback for highly viscous materials**
Motorisierte handgehaltene Injektionsvorrichtung mit haptischer Rückmeldung für hochviskose Materialien
Dispositif d'injection à main motorisé à rétroaction haptique pour matériaux très visqueux

(43) Date of publication of application: 16.05.2007
(73) Proprietor: Synthes GmbH, 4436 Oberdorf (CH)
(72) Inventor: LOEFFEL, Mario, CH-3072 Ostermundigen (CH); PAPPAS, Ion, HU-1118 Budapest (HU); NOLTE, Lutz-Peter, CH-3818 Grindelwald (CH)
(74) Representative: Lusuardi, Werther
(86) International application number: PCT/CH2004/000543
(87) International publication number: WO 2006/024177

(56) References cited:
- US-A- 5 762 458
- US-A1- 2003 036 762

## Description

The invention relates to a hand-held motorized injection device with haptic feedback for highly viscous materials .

In certain surgical interventions, in particular on the spine, such as vertebroplasty, kyphoplasty and spinal disc nucleus replacement, it is required to inject highly viscous materials (e.g. bone cement) into bony or cartilaginous structures. Currently, the injection is performed either manually with a traditional syringe or by using screw-type systems., In non-spinal applications cement delivery is sometimes performed using gun-type injection devices.

When using traditional syringes for injecting highly viscous materials, such as bone cement in vertebroplasty or kyphoptasty, forces up to 200 N have to be exerted by the operator's thumb. Therefore, small pressure changes are hard to perceive.

Systems that generate higher pressures are typically based on a screw paradigm: The user screws the plunger into a threaded cylinder in order to press out the cement. With screw-type systems, much higher pressure can be generated but the drawback is that the haptic feedback is eliminated.

From US-B2-6,425,897 OVERES ET AL. a pistol for the pressing out of bone cement with an attachable cement syringe is known. The ejection of the bone cement is based on a hydraulic principle giving no haptic, i.e. tactile feedback to the person actuating the pistol.

US-A-5,762,458 WANG ET AL. discloses a device according to the preamble of claim 1.

On this point, the invention intends to provide remedial measures. The invention is based on the objective of providing an injection device which is able to eject a highly viscous material under high pressure and simultaneously offering a haptic feedback to the operator.

The invention solves the posed problem with a device that displays the features of claim 1.

In the preferred embodiment, the novel injection device for viscous materials, in particular bone cement, consists of a motorized injection module coupled with a syringe-like master driver with haptic feedback. In this particular embodiment of the device we use a DC-motor with gears to drive the syringe plunger and magneto-rheological fluids to generate the haptic feedback.

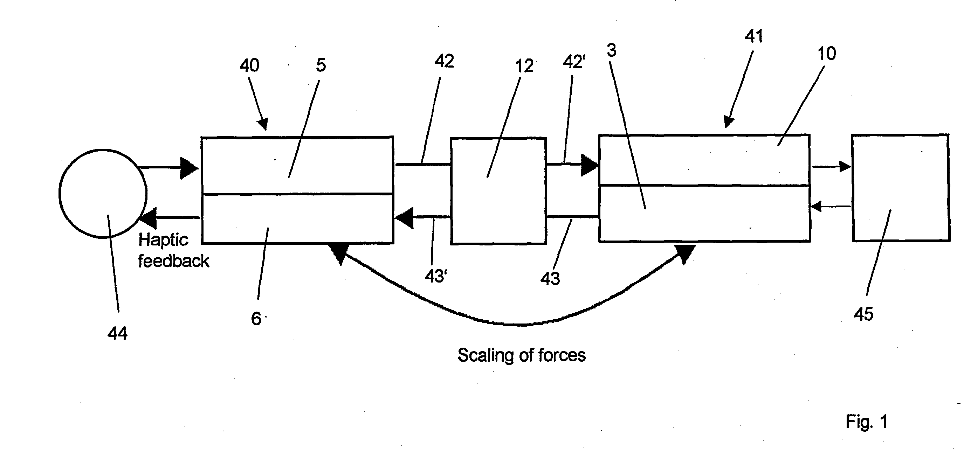

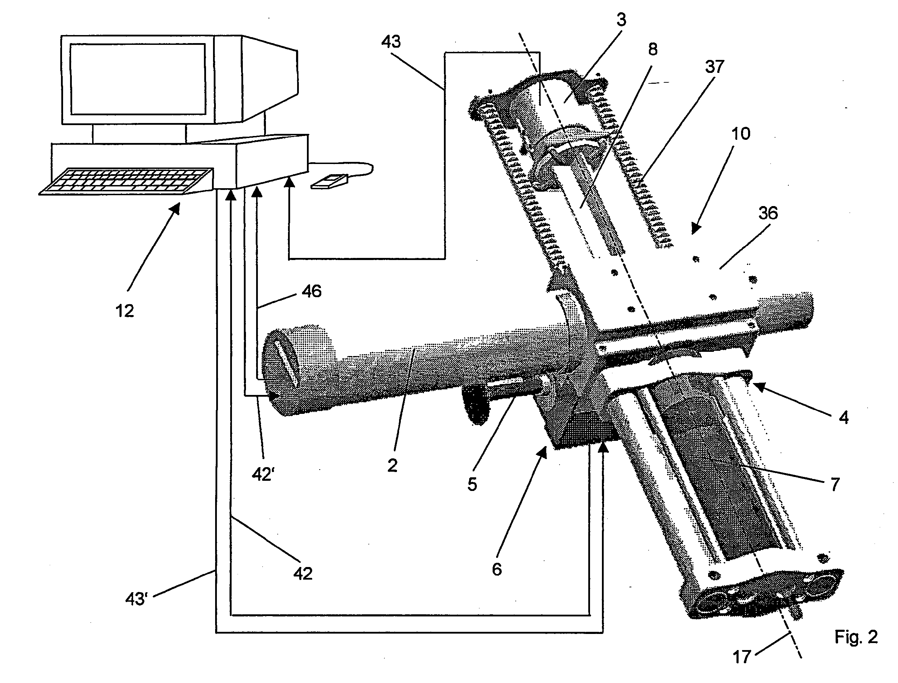

The system is best described as a master-slave setup (Fig. 1). The user's action on the master driver with the haptic interface is translated into an advancement or retraction of the motorized syringe plunger. The pressure measured in the syringe or the force measured on the syringe plunger is fed back to the haptic unit of the master driver, providing tactile feedback to the operator (Fig. 2).

The device is computer controlled, therefore parameters such as measured forces and injection speed are freely scalable. For example small pressure changes can be amplified on the haptic feedback. Additionally, signal processing and control algorithms can be introduced for the detection and prevention of potential errors or dangers.

The advantages achieved by the invention are essentially the following:
1. Computer-controlled injection offering the possibility of controlling various injection parameters (e.g. constant pressure, constant flux) and preprocessing or filtering of operator input to avoid errors;
2. Real-time, scalable haptic feedback based on measured pressure changes;
3. Real-time calculation of viscosity of the highly viscous material;
4. Ability to reverse and pull the syringe plunger resulting in an emergency stop or even a suction effect;
5. Injection parameter logging for documentation purposes and experimental studies; and
6. Reusable, sterilizable device lowering the costs on the long run compared to disposable screw-type systems.

The advantages of known devices, i.e.:
- high pressure injection of viscous materials; and
- the use of standard syringes
may be retained by the injection device according to the invention.

Additional advantageous embodiments of the invention are defined in the subclaims.

In a preferred embodiment the master component comprises a haptic unit which is provided, with a master driver. This offers the advantage that the master driver which is actuated by the operator may be used to emit the actuation information applied by the operator to the master component as well as to provide the operator with the haptic feedback related to the sensor information.

In another embodiment the device further comprises a control unit which allows a scaling of forces between the master component and the slave component. Herewith, the advantage is achieved that the force applied on a surgical apparatus by the slave unit may be increased relative to the force applied to the master driver by the operator's hand and the forces measured by the sensor and that are fed back to the master driver in order to provide the operator with a tactile feedback may be reduced.

In yet another embodiment the device comprises a drive unit with a motor having a power transmission and being configured to displace a plunger of a syringe attached to the injection device. Furthermore, the sensor may be apt to emit sensor information related to the measured loads acting on a plunger of a syringe attached to the injection device.

The motor may be driven by electric, hydraulic or pneumatic forces. Furthermore, the sensor may be realized through a load sensor between the actuator and the plunger of the syringe, or through measurement of the motor torque e.g. by measuring the motor electric current or through measurement of the pressure in the syringe. Preferably, the motor is autoclavable.

In a further embodiment the haptic unit is configured to convert the electrical output signal emitted by the control unit into a mechanical resistance opposing displacement of the master driver. This offers the advantage that the forces to be excerted by the operator's thumb, which may be up to 200 N during the injection of highly viscous materials, such as bone cement are reduced by retaining the ability of small pressure changes being tactile for the operator.

In yet a further embodiment the haptic unit comprises a magneto-rheological unit. Compared to other haptic units such as electro-mechanical units, memory metal units or electro-active polymer units the magneto-rheological unit shows the advantages of:
- a fast reaction time (10ms); faster than electro-mechanical or memory metal units;
- being sterilizable (difficult to achieve with an electro-mechanical unit);
- moving-parts free;
- moderate voltages required (much lower than with electro-active polymer unit);
- high momentum compared to electro-active polymer unit and;
- accurately controllable (difficult to achieve with a memory material unit).

In another preferred embodiment the magneto-rheological unit comprises a housing with a first end, a second end and a coaxial fluid chamber with at least one opening at the first end as leadthrough for the master driver. Furthermore, the master driver preferably comprises a plunger with a plunger rod both being arranged coaxially to the longitudinal axis of the housing, whereby the plunger is provided with at least one coaxial coil. The magneto-rheological unit comprises a magneto-rheological fluid which is preferably based on a hydrocarbon oil.

In yet another embodiment the fluid chamber has a hollow cylindrical inner wall having an interior diameter D, and the plunger has an outer diameter d < D, in order to allow a fluid gap between the plunger of the master driver and the fluid chamber. The fluid gap may have a width W of between 0,1 mm and 1,0 mm, preferably between 0,3 mm and 0,7 mm.

Preferably, the at least one coil generates a magnetic field having lines of magnetic flux within the fluid gap that are extending parallel to the longitudinal axis of the housing, whereby the magnetic flux depends on the electrical output signal of the control unit.

In a further embodiment the plunger of the master driver comprises two coils arranged axially adjoining and having opposite directions of magnetic flux. This offers the advantage of a stronger magnetic field in the fluid gap. The magnetic field shifts from the center of the iron core towards the middle gap.

Preferably, each of the two coils comprises an iron core being provided with a first, respectively second iron flange of an axial length L₁; L₂. The first and second iron flange radially extending until the outer diameter of the coil at the free end of the coil. Between the two coils a third iron flange is arranged which has an axial length L₃. Preferably, the length L = L₁ + L₂ + L₃ is 1 ≤ L ≤ 5 mm.

In another embodiment the injection device comprises a syringe receiving means attached to the drive unit and being apt to receive a syringe containing the highly viscous material. Furthermore, the device may comprise a syringe.

In yet another embodiment the control unit is apt to receive position measurement signals of the plunger of the master driver and of the motor as well.

The invention and additional configurations of the invention are explained in even more detail with reference to the partially schematic illustration of several embodiments.

Shown are:
Fig. 1 schematically, a diagram for a master-slave setup;
Fig. 2 perspectively, an embodiment of the injection device according to the invention;
Fig. 3 an exploded view of a the embodiment according to Fig. 2;
Fig. 4 schematically, the injection device according to the invention applied to a patient;
Fig. 5 a longitudinal cross section of the haptic unit; and
Fig. 6 an enlarged section according to the marked field A in Fig. 5.

In Fig. 1 the master-slave setup is schematically shown, whereby the master component 40 is being manually controlled by the human operator 44 by means of a master driver 5 (Fig. 2) which allows to actuate the slave component 41. Furthermore, the master component 40 is apt to provide the operator with a haptic feedback by means of a haptic unit 6. The slave component 41 comprises a drive unit 10 actuating a surgical apparatus 45, e.g. a syringe 7 (Fig. 2) or other apparatus and is being controlled by the master component 40. Furthermore, the slave component 41 is provided with a sensor 3 apt to measure forces or pressure applied to the surgical apparatus 45 through the drive unit 10. The actuation information 42;42' emitted by the master component 40 as well as the sensor information 43;43' emitted through the sensor 3 are being scaled by a control unit 12 and transmitted between the master component 40 and the slave component 41 such that a scaling of forces is performed between the master component 40 and the slave component 41. Particularly, the scaling of forces is performed between the actuation information 42 emitted by the master component 40 and sent to the control unit 12 and the scaled actuation information 42' sent from the control unit 12 to the master component 41 as well as between the sensor information 43 sent from the sensor 3 to the control unit 12 and the scaled sensor information 43' sent from the control unit 12 to the haptic unit 6. The human operator 44 pushes or pulls the master driver 5 (Fig. 2) whereby the information of the displacement, i.e. position and speed are fed into the control unit 12 which converts the user's motion of the master driver 5 into a freely programmable, scaled actuation information 42 transmitted to the drive unit 10. The resulting force or pressure effected to a surgical apparatus 45 by the drive unit 10 is calculated from the force measurement by the sensor 3 and routed back to the control unit 12. A freely programmable tactile feedback is generated on the master driver 5 (Fig. 2) based on the sensor information 43. The human operator 44 perceives active changes in the resistance of the master driver 5 (Fig. 2) being related to the resistance opposing the force applied to the surgical apparatus 45 by means of the drive unit 10.

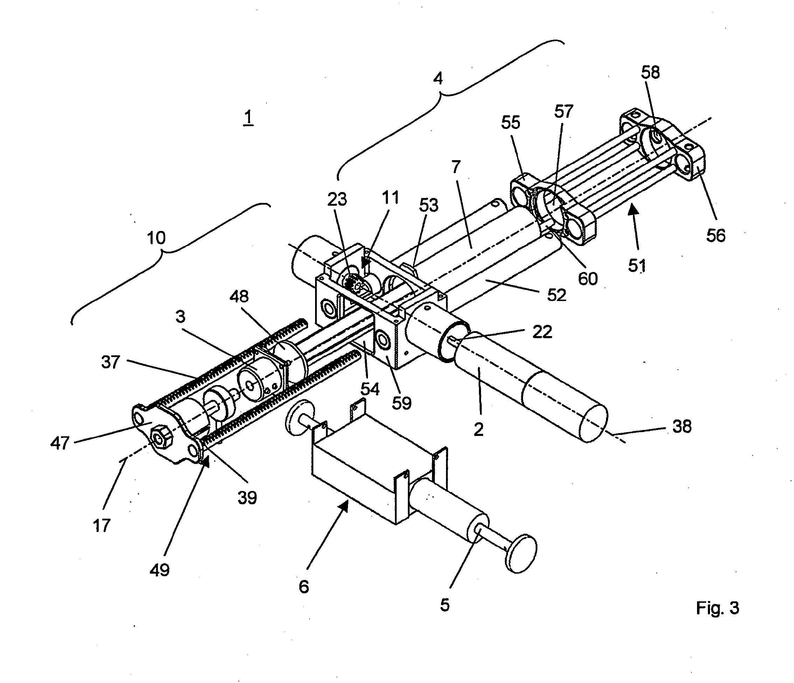

Fig. 2 and 3 show an embodiment of the device configured as injection device 1 for highly viscous materials. The drive unit 10 comprises a central axis 17 corresponding to the axis of the syringe 7 which is attached to the drive unit 10 by means of a syringe receiving means 4. Furthermore, the drive unit 10 comprises a motor 2 having a shaft 22 with an axis 38 perpendicular to the longitudinal axis 17 of the drive unit 10, a power transmission 11 which includes a gearwheel 23 being coaxially attached to the shaft 22 and being in engagement with a toothed rack 37 arranged and displaceable parallel to the longitudinal axis 17 of the drive unit 10. A second toothed rack 37 also being extending and displaceable parallel to the longitudinal axis 17 is connected to the drive unit 10. The second toothed rack 37 is in engagement with a second gearwheel 23 which is situated coaxial to the axis 38 of the shaft 22 and opposite with respect to the central axis 17. Depending on the operator being left - or right handed the motor 2 may be fastened on either side of the gear box 59 such that the gearwheel 23 engages either the first or second tooth rack 37a;37b. The two toothed racks 37 are connected at their rear ends 39 by a end plate 47 and form an actuator 49 driven by the motor 2 and configured to press on to the rear end 48 of the plunger 8 to displace the plunger 8 of the syringe 7 attached to the drive unit 10. The sensor 3 is attached to the end plate 47 intermediate to the rear end 48 of the plunger 8 and is apt to emit sensor information 43 (Fig. 2) related to the measured loads effected on the plunger 8 through the actuator 49. Furthermore, the drive unit 10 comprises a syringe receiving means 4 attached to the drive unit 10 and apt to receive a syringe 7 containing the highly viscous material. As shown in fig. 3 the syringe receiving means 4 comprise a cage 51 and two posts 52 extending parallel to the central axis 17 of the drive unit 10 and being attached to the drive unit 10. The syringe 7 may be placed between the two posts 52 such that the rear end 53 of the chamber of the syringe 7 rests at the wall of the gear box 59 while plunger rod of the plunger 8 of the syringe 7 is lead through a through opening 54 coaxially penetrating the gear box 59 of the drive unit 10 and so that the rear end 48 of the plunger 8 of the syringe 7 may be attached to the actuator 49. After inserting the syringe 7 between the posts 52 the cage 51 is mounted and fastened to the two posts 52. The cage 51 has two end plates 55;56 arranged perpendicularly to the central axis 17, a first coaxial bore 57 for the body of the syringe 7 in the first end plate 55 adjacent to the drive unit 10 and a second coaxial bore 58 for the outlet 60 of the syringe 7. Such the syringe 7 is rigidly fixed to the gear box 59 of the drive unit 10 while the plunger 8 of the syringe 7 may be displaced by means of the actuator 49 of the drive unit 10.

Furthermore, the control unit 12 further comprises a motor feedback 46 in order to receive position measurement signals of the plunger 25 of the master driver 5 and the motors 2. The motor feedback 46 as well as the actuator information 42 and the sensor information 43 may be transmitted through electrical wiring or wireless.

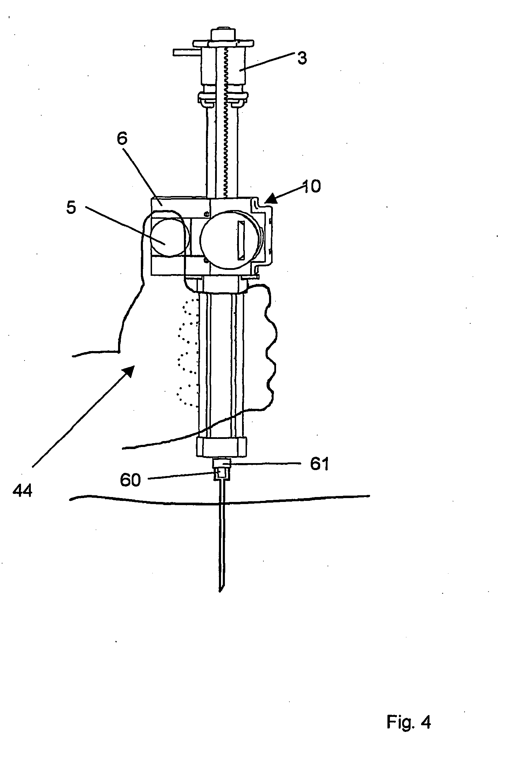

Fig. 4 depicts the use of the device 1. The operator 44 pushes or pulls the master driver 5 whereby the information of the displacement, i.e. position and speed are fed into the control unit 12 (Fig. 2) which converts the operator's motion of the master driver 5 into a freely programmable, scaled motion on the plunger 8 (Fig. 3) by means of the drive unit 10. The resulting pressure in the syringe 7 (Fig. 2) is calculated from the force measurement by the sensor 3 and routed back to the control unit 12 (Fig. 2). A freely programmable tactile feedback is generated on the master driver 5 based on the sensor information 43 (Fig. 2) by means of the haptic unit 6. The operator perceives active changes in the resistance of the master driver 5 being related to the resistance of the plunger 8 (Fig. 3) within the syringe 7. Furthermore, the outlet 60 of the syringe 7 comprises a valve 61.

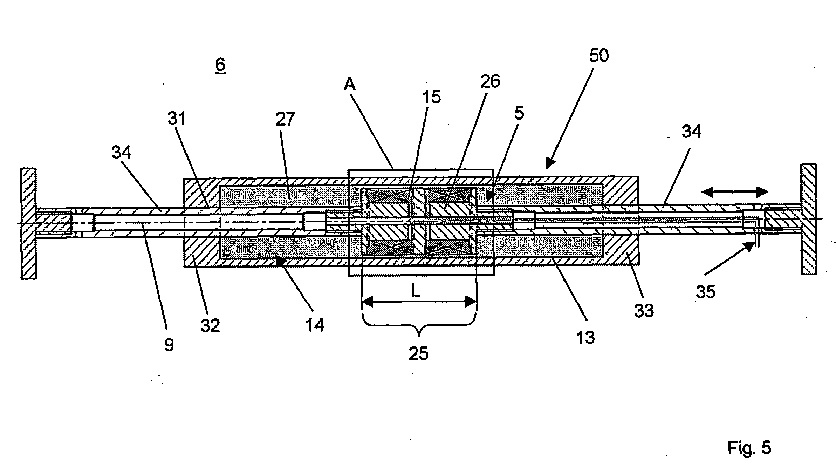

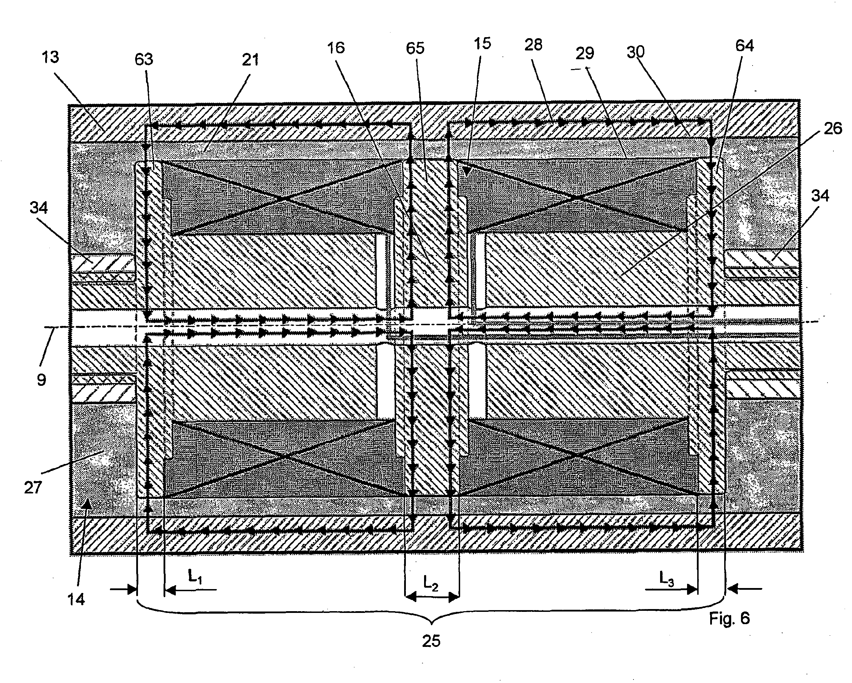

Fig. 5 and 6 depict the haptic unit 6 of the embodiment according to Fig. 2. The haptic unit 6 is realized through a magneto-rheological unit 50 which essentially comprises a housing 13 having a longitudinal axis 9 and being provided with a hollow cylindrical fluid chamber 14 arranged concentrical to the longitudinal axis 9 and a plunger 25 being displaceable within the fluid chamber 14 parallel to the longitudinal axis 9. The fluid chamber 14 has a coaxial opening 31 at the first and second axial end 32;33 of the housing 13. The master driver 5 is realized through an axially displaceable plunger 25 which is provided with a plunger rod 34 at each of its axial ends. Two plunger rods 34 coaxially extend through the openings 31 such that the operator may manually displace the plunger 25 in each axial direction by pushing or pulling on of the plunger rods 34. The fluid chamber 14 in the housing 13 is filled with a magneto-rheological fluid 27 (MR-fluid) which consists of a carrier fluid and roughly 30 volume-percent nano-sized particles. When a magnetic field is applied to the MR-fluid by means of the coils 15, the particles align in the direction of the lines 28 of the magnetic field, thus increasing the viscosity of the MR-fluid 27. The plunger 25 is equipped with a dual copper coil 15 wound around a iron core 26, forming an electromagnet in the plunger 25. The dual copper coil 15 is electrically connected to a power source (not shown) by means of the wires 35. When the electromagnet is turned off, the master driver 5 can be moved freely in the fluid chamber 14 - the MR-fluid 27 flows freely through the fluid gap 21 between the lateral surface 29 of the plunger 25 and the wall 30 of the fluid chamber 14. When the electromagnet is turned on, a magnetic field is generated which has magnetic field lines 28 extending parallel to the longitudinal axis 9 of the fluid chamber 14 and the viscosity of the MR-fluid in the fluid gap 21 increases, which results in a higher resistive force opposing the displacement of the master driver 5. The change of viscosity may be controlled by regulating the electric current running through the coils 15. A higher electric current produces a stronger magnetic field, which leads to the production of stronger metal particle chains in the MR-fluid. The two iron cores 26 are provided with a first, respectively second iron flange 63;64 of an axial length L₁; L₂, and being radially extending until the outer diameter of the coil 15 at the free end of the plunger 25. Furthermore, the plunger 25 comprises a third iron flange 65 situated axially between the two coils 15 an having an axial length L₃.

## Claims

1. Device for driving a surgical apparatus (45) and being configured in a master-slave setup with
A) a slave component (41) comprising
A1) a drive unit (10) apt to actuate a surgical apparatus (45) connected to it; and
A2) a sensor (3) apt to emit sensor information (43) related to the resistance opposing the actuation of the drive unit (10); and
B) a master component (40) allowing a haptic feedback related to the resistance detected by the sensor (3),
**characterised in that**
C) the device is configured as an injection device (1) for highly viscous materials.

2. Device according to claim 1, **characterized in that** the master component (40) allows a scalable haptic feedback.

3. Device according to claim 1 or 2, **characterized in that** the master component (40) comprises a haptic unit (6) being provided with a master driver (5).

4. Device according to one of the claims 1 to 3, **characterized in that** it further comprises a control unit (12) allowing a scaling of forces between the master component (40) and the slave component (41).

5. Device according to one of the claims 1 to 4, **characterized in that** it comprises a drive unit (10) with a central axis (17) and a motor (2) having a power transmission (11) being configured to displace a plunger (8) of a syringe (7) attached to the injection device (1).

6. Device according to claim 5, **characterized in that** the sensor (3) is apt to emit sensor information (43) related to the measured loads acting on a plunger (8) of a syringe (7) attached to the injection device (1).

7. Device according to claim 5 or 6, **characterized in that** the haptic unit (6) is apt to convert the electrical output signal emitted by the control unit (12) into a mechanical resistance opposing displacement of the master driver (5).

8. Injection device (1) according to claim 3 , **characterized in that** the haptic unit (6) comprises a magneto-rheological unit (50).

9. Injection device (1) according to claim 8, **characterized in that** the magneto-rheological unit (50) comprises a housing (13) with a longitudinal axis (9), a first end (32), a second end (33) and a coaxial fluid chamber (14) with at least one opening (31) at the first end (32) as leadthrough for the master driver (5).

10. Injection device (1) according to claim 9, **characterized in that** the master driver (5) comprises a plunger (25) with a plunger rod (34) both being arranged coaxially to the longitudinal axis (9).

11. injection device (1) according to claim 10, **characterized in that** the plunger (25) of the master driver (5) is provided with at least one coaxial coil (15).

12. Injection device (1) according to one of the claims 8 to 11, **characterized in that** the magneto-rheological unit (50) comprises a magneto-rheological fluid which is based on a hydrocarbon oil.

13. Injection device (1) according to one of the claims 9 to 12, wherein the fluid chamber (14) has a hollow cylindrical inner wall (30) having an interior diameter D, and the plunger (25) of the master driver (5) has an outer diameter d < D, in order to allow a fluid gap (21) between the plunger (25) of the master driver (5) and the fluid chamber (14).

14. Injection device (1) according to claim 13, wherein the fluid gap (21) has a width W of between 0,1 mm and 1,0 mm, preferably between 0,3 mm and 0,7 mm.

15. Injection device (1) according to one of the claims 11 to 14, wherein the at least one coil (15) generates a magnetic field having lines of magnetic flux within the fluid gap (21) that are extending parallel to the longitudinal axis (9).

16. Injection device (1) according to claim 15, wherein the magnitude of the magnetic flux is depending on the electrical output signal of the control unit (12).

17. Injection device (1) according to one of the claims 10 to 16, wherein the plunger (25) of the master driver (5) comprises two coils (15) arranged axially adjoining and having opposite directions of magnetic flux.

18. Injection device (1) according to claim 17, wherein each of the two coils (15) comprises an iron core (26) being provided with a first, respectively second iron flange (63;64) of an axial length L₁;L₂, and which is radially extending until the outer diameter of the coil (15) at the free end of the coil (15) and wherein the plunger (25) comprises a third iron flange (65) situated axially between the two coils (15) an having an axial length L₃.

19. Injection device (1) according to claim 18, wherein the length L = L₁ + L₂ + L₃ is 1 ≤ L ≤ 5 mm.

20. Injection device (1) according to one of the claims 1 to 19, wherein it comprises a syringe receiving means (4) attached to the drive unit (10) and being apt to receive a syringe (7) containing the highly viscous material;

21. Injection device (1) according to one of the claims 1 to 20, wherein it further comprises a syringe (7).

22. Injection device (1) according to one of the claims 5 to 21, wherein the motor (2) is autoclavable.

23. Injection device (1) according to claims 3 and 5, wherein the control unit (12) is apt to receive position measurement signals of the plunger (25) of the master driver (5) and the motor (2).

## Patentansprüche

1. Vorrichtung zum Antreiben einer chirurgischen Vorrichtung (45), welche als Master-Slave-Anordnung konfiguriert ist, mit
A) einer Arbeitskomponente (41) umfassend
A1) eine Antriebseinheit (10), welche zum Antrieb einer mit ihr verbundenen chirurgischen Vorrichtung (45) geeignet ist; und
A2) einen Sensor (3), der zur Abgabe von Sensor-Information (43) betreffend den Widerstand, der dem Antrieb der Antriebseinheit (10) entgegenwirkt, geeignet ist; und
B) einer Leitkomponente (40), die eine haptische Rückmeldung betreffend den durch den Sensor (3) detektierten Widerstand ermöglicht,
**dadurch gekennzeichnet, dass**
C) die Vorrichtung als Injektionsvorrichtung (1) für hochviskose Materialien konfiguriert ist.

2. Vorrichtung nach Anspruch 1, **dadurch gekennzeichnet, dass** die Leitkomponente (40) eine skalierbare haptische Rückmeldung ermöglicht.

3. Vorrichtung nach Anspruch 1 oder 2, **dadurch gekennzeichnet, dass** die Leitkomponente (40) eine mit einem Leitantrieb (5) ausgestattete haptische Einheit (6) umfasst.

4. Vorrichtung nach einem der Ansprüche 1 bis 3, **dadurch gekennzeichnet, dass** sie zusätzlich eine Steuerungseinheit (12) umfasst, die eine Skalierung von Kräften zwischen der Leitkomponente (40) und der Arbeitskomponente (41) ermöglicht.

5. Vorrichtung nach einem der Ansprüche 1 bis 4, **dadurch gekennzeichnet, dass** sie eine Antriebseinheit (10) mit einer Zentralachse (17) und einem Motor (2) mit einer Kraftübertragung (11) umfasst, die dazu ausgebildet ist, einen Kolben (8) einer an der Injektionsvorrichtung (1) angebrachten Spritze (7) zu verschieben.

6. Vorrichtung nach Anspruch 5, **dadurch gekennzeichnet, dass** der Sensor (3) dazu geeignet ist, Sensor-Information (43) abzugeben, die in Beziehung zu den gemessenen Belastungen steht, die auf einen Kolben (8) einer an der Injektionsvorrichtung (1) angebrachten Spritze (7) wirken.

7. Vorrichtung nach Anspruch 5 oder 6, **dadurch gekennzeichnet, dass** die haptische Einheit (6) dazu geeignet ist, das von der Steuerungseinheit (12) abgegebene elektrische Ausgangssignal in einen mechanischen Widerstand umzuwandeln, der der Verschiebung des Leitantriebs (5) entgegenwirkt.

8. Injektionsvorrichtung (1) nach Anspruch 3, **dadurch gekennzeichnet, dass** die haptische Einheit (6) eine magneto-rheologische Einheit (50) umfasst.

9. Injektionsvorrichtung (1) nach Anspruch 8, **dadurch gekennzeichnet, dass** die magneto-rheologische Einheit (50) ein Gehäuse (13) mit einer Längsachse (9), einem ersten Ende (32), einem zweiten Ende (33) und einer koaxialen Flüssigkeitskammer (14) mit mindestens einer Öffnung (31) am ersten Ende (32) als Durchgang für den Leitantrieb (5) umfasst.

10. Injektionsvorrichtung (1) nach Anspruch 9, **dadurch gekennzeichnet, dass** der Leitantrieb (5) einen Kolben (25) mit einer Kolbenstange (34) umfasst, wobei beide koaxial zur Längsachse (9) angeordnet sind.

11. Injektionsvorrichtung (1) nach Anspruch 10, **dadurch gekennzeichnet, dass** der Kolben (25) des Leitantriebs (5) mit mindestens einer koaxialen Spule (15) ausgestattet ist.

12. Injektionsvorrichtung (1) nach einem der Ansprüche 8 bis 11, **dadurch gekennzeichnet, dass** die magneto-rheologische Einheit (50) eine magneto-rheologische Flüssigkeit umfasst, die auf einem Kohlenwasserstofföl basiert.

13. Injektionsvorrichtung (1) nach einem der Ansprüche 9 bis 12, **dadurch gekennzeichnet, dass** die Flüssigkeitskammer (14) eine hohlzylindrische Innenwand (30) mit einen Innendurchmesser D hat, und der Kolben (25) des Leitantriebs (5) einen Außendurchmesser d < D hat, um einen Spalt (21) für eine Flüssigkeit zwischen dem Kolben (25) des Leitantriebs (5) und der Flüssigkeitskammer (14) zu ermöglichen.

14. Injektionsvorrichtung (1) nach Anspruch 13, **dadurch gekennzeichnet, dass** der Spalt (21) für eine Flüssigkeit eine Weite W von zwischen 0,1 mm und 1.0 mm, vorzugsweise zwischen 0,3 mm und 0,7 mm hat.

15. Injektionsvorrichtung (1) nach einem der Ansprüche 11 bis 14, **dadurch gekennzeichnet, dass** die mindestens eine Spule (15) ein Magnetfeld erzeugt, das Linien magnetischen Flusses innerhalb des Spalts (21) für eine Flüssigkeit hat, die sich parallel zur Längsachse (9) erstrecken.

16. Injektionsvorrichtung (1) nach Anspruch 15, **dadurch gekennzeichnet, dass** die Größe des magnetischen Flusses von dem elektrischen Ausgangssignal der Steuerungseinheit (12) abhängig ist.

17. Injektionsvorrichtung (1) nach einem der Ansprüche 10 bis 16, **dadurch gekennzeichnet, dass** der Kolben (25) des Leitantriebs (5) zwei Spulen (15) umfasst, die axial angrenzend angeordnet sind und entgegengesetzte Richtungen des magnetischen Flusses haben.

18. Injektionsvorrichtung (1) nach Anspruch 17, **dadurch gekennzeichnet, dass** jede der zwei Spulen (15) einen Eisenkern umfasst (26), der mit einem ersten, beziehungsweise zweiten Eisenflansch (63; 64) einer axialen Länge L₁; L₂ ausgestattet ist und der sich radial bis zum Aussendurchmesser der Spule (15) am freien Ende der Spule (15) erstreckt und wobei der Kolben (25) einen dritten Eisenflansch (65) umfasst, der axial zwischen den zwei Spulen (15) angeordnet ist und der eine axiale Länge L₃ hat.

19. Injektionsvorrichtung (1) nach Anspruch 18, **dadurch gekennzeichnet, dass** die Länge L = L₁ + L₂ + L₃, 1 ≤ L ≤ 5 mm beträgt.

20. Injektionsvorrichtung (1) nach einem der Ansprüche 1 bis 19, **dadurch gekennzeichnet, dass** sie eine Aufnahmeeinrichtung (4) für eine Spritze umfasst, die an der Antriebseinheit (10) befestigt ist und dazu geeignet ist, eine Spritze (7) aufzunehmen, die das hochviskose Material enthält.

21. Injektionsvorrichtung (1) nach einem der Ansprüche 1 bis 20, **dadurch gekennzeichnet, dass** sie zusätzlich eine Spritze (7) umfasst.

22. Injektionsvorrichtung (1) nach einem der Ansprüche 5 bis 21, **dadurch gekennzeichnet, dass** der Motor (2) autoklavierbar ist.

23. Injektionsvorrichtung (1) nach den Ansprüchen 3 und 5, **dadurch gekennzeichnet, dass** die Steuerungseinheit (12) dazu geeignet ist, Positionsmesssignale des Kolbens (25) des Leitantriebs (5) und des Motors (2) zu empfangen.

## Revendications

1. Dispositif permettant de commander un appareil chirurgical (45) et qui est configuré suivant une programmation maître-esclave comprenant :
A) une composante esclave (41) comportant
A1) une unité de commande (10) capable d'actionner un appareil chirurgical (45) connecté à elle ; et
A2) un capteur (3) capable d'émettre une information de capteur (43) se rapportant à la résistance s'opposant à l'action de l'unité de commande (10); et
B) une composante maître (40) permettant une rétroaction haptique se rapportant à la résistance détectée par le capteur (3),
**caractérisé en ce que**
C) le dispositif est configuré sous la forme d'un dispositif d'injection (1) destiné à des matériaux fortement visqueux.

2. Dispositif selon la revendication 1, **caractérisé en ce que** la composante maître (40) permet une rétroaction haptique pouvant être étalonné.

3. Dispositif selon la revendication 1 ou 2, **caractérisé en ce que** la composante maître (40) comprend une unité haptique (6) dotée d'un dispositif de commande maître (5).

4. Dispositif selon l'une des revendications 1 à 3, **caractérisé en ce qu'**il comprend, de plus, une unité de commande (12) permettant un étalonnage des forces entre la composante maître (40) et la composante esclave (41).

5. Dispositif selon l'une des revendications 1 à 4, **caractérisé en ce qu'**il comprend une unité de commande (10) dotée d'un axe central (17) et d'un moteur (2) ayant une transmission de puissance (11) configurée pour déplacer un piston (8) d'une seringue (7) fixée au dispositif d'injection (1).

6. Dispositif selon la revendication 5, **caractérisé en ce que** le capteur (3) est apte à émettre une information de capteur (43) se rapportant aux charges mesurées agissant sur un piston (8) d'une seringue (7) fixée au dispositif d'injection (1).

7. Dispositif selon la revendication 5 ou 6, **caractérisé en ce que** l'unité haptique (6) est capable de convertir le signal électrique de sortie émis par l'unité de commande (12) en une résistance mécanique s'opposant au déplacement du dispositif de commande maître (5).

8. Dispositif d'injection (1) selon la revendication 3, **caractérisé en ce que** l'unité haptique (6) comprend une unité magnéto-rhéologique (50).

9. Dispositif d'injection (1) selon la revendication 8, **caractérisé en ce que** l'unité magnéto-rhéologique (50) comprend un logement (13) présentant un axe longitudinal (9), une première extrémité (32), une seconde extrémité (33) et une chambre de fluide coaxiale (14) dotée d'au moins une ouverture (31) au niveau de la première extrémité (32) en tant que passage pour le dispositif de commande maître (5).

10. Dispositif d'injection (1) selon la revendication 9, **caractérisé en ce que** le dispositif de commande maître (5) comporte un piston (25) doté d'une tige de piston (34) tous deux étant agencés de façon coaxiale à l'axe longitudinal (9).

11. Dispositif d'injection (1) selon la revendication 10, **caractérisé en ce que** le piston (25) du dispositif de commande maître (5) est équipé d'au moins une bobine coaxiale (15).

12. Dispositif d'injection (1) selon l'une des revendications 8 à 11, **caractérisé en ce que** l'unité magnéto-rhéologique (50) comprend un fluide magnéto-rhéologique qui est à base d'une huile hydrocarbonée.

13. Dispositif d'injection (1) selon l'une des revendications 9 à 12, dans lequel la chambre de fluide (14) comporte une paroi intérieure cylindrique creuse (30) présentant un diamètre intérieur D, et le piston (25) du dispositif de commande maître (5) présente un diamètre extérieur d < D, afin de rendre possible un espace destiné à un fluide (21) entre le piston (25) du dispositif de commande maître (5) et la chambre de fluide (14).

14. Dispositif d'injection (1) selon la revendication 13, dans lequel l'espace destiné à un fluide (21) présente une largeur W comprise entre 0,1 mm et 1,0 mm, de préférence entre 0,3 mm et 0,7 mm.

15. Dispositif d'injection (1) selon l'une des revendications 11 à 14, dans lequel la, au moins une, bobine (15) génère un champ magnétique ayant des lignes de flux magnétique à l'intérieur de l'espace destiné à un fluide (21) qui s'étendent parallèlement à l'axe longitudinal (9).

16. Dispositif d'injection (1) selon la revendication 15, dans lequel l'intensité du flux magnétique dépend du signal électrique de sortie de l'unité de commande (12).

17. Dispositif d'injection (1) selon l'une des revendications 10 à 16, dans lequel le piston (25) du dispositif de commande maître (5) comprend deux bobines (15) disposées axialement de façon adjacente et présentant des directions opposées de flux magnétique.

18. Dispositif d'injection (1) selon la revendication 17, dans lequel chacune des deux bobines (15) comporte un noyau de fer (26) pourvu, respectivement, d'une première et d'une deuxième joues de fer (63 ; 64) d'une longueur axiale L₁ ; L₂, et qui s'étendent de façon radiale jusqu'au diamètre extérieur de la bobine (15) au niveau de l'extrémité libre de la bobine (15) et dans lequel le piston (25) comporte une troisième joue de fer (65) située axialement entre les deux bobines (15) et présentant une longueur axiale L₃.

19. Dispositif d'injection (1) selon la revendication 18, dans lequel la longueur L = L₁ + L₂ + L₃ est 1 ≤ L ≤ 5 mm.

20. Dispositif d'injection (1) selon l'une des revendications 1 à 19, dans lequel il est compris des moyens de réception de seringue (4) fixés à l'unité de commande (10) et aptes à recevoir une seringue (7) contenant le matériau fortement visqueux.

21. Dispositif d'injection (1) selon l'une des revendications 1 à 20, dans lequel il est compris, de plus, une seringue (7).

22. Dispositif d'injection (1) selon l'une quelconque des revendications 5 à 21, dans lequel le moteur (2) est autoclavable.

23. Dispositif d'injection (1) selon les revendications 3 et 5 dans lequel l'unité de commande (12) est apte à recevoir des signaux de mesure de positionnement du piston (25) du dispositif de commande maître (5) et du moteur (2).
